# EUROPEAN PATENT APPLICATION

(11) **EP 1 842 525 A2**
(43) Date of publication of application: **10.10.2007**
(21) Application number: 07005960.5
(22) Date of filing: 22.03.2007
(51) Int. Cl.: A61K 8/19, A61K 8/11, A61Q 17/00, A45D 29/00

(54) **Antibacterial gel coating and pedicure spa with antibacterial function**

(30) Priority: 22.03.2006 US 386633
(71) Applicant: Kiss Nail Products Inc., Port Washington NY 11050 (US)
(72) Inventor: Kim, Dae Jin, Port Washington, NY 11050 (US)
(74) Representative: Epping - Hermann - Fischer

(57) **Abstract**

A pedicure spa with antibacterial function includes a tub having a surface and a gel coating on the surface of the tub. The gel coating includes an industrially applicable gel coating mixed with silver nano-particles having a diameter in the range of approximately 10 - 35 nm such that a concentration of the silver nano-particles is approximately 50 - 200 ppm.

## Description

### BACKGROUND OF THE INVENTION

### 1. Field of the Invention

The present invention relates generally to a pedicure equipment and an antibacterial gel coating. More particularly, the present invention relates to a pedicure spa with antibacterial function and an antibacterial gel coating for top-coating a tub or sink of a pedicure spa.

### 2. Description of the Related Art

Receiving a pedicure has become a popular leisure activity, especially among female consumers, and pedicure spas, on which pedicure is performed, have been developed and used for some time.

In a typical pedicure session, a customer sits on a chair of a pedicure spa, with her feet submerged in warm water and soapsuds in a tub or sink of the pedicure spa. Additives such as olive oil and bath salts may be added to the contents of the tub. After the feet have been submerged in the warm soapsuds and water for a predetermined period of time, the customer's feet are placed on a footrest of the pedicure spa where the toenails may be trimmed and painted and other related services may be performed on the feet, toes, and/or nails of the customer.

After the pedicure session is completed, the water and soapsuds are drained from the tub of the pedicure spa, and the tub is rinsed or washed with water to ensure that the pedicure spa is clean and ready for the next customer.

Various bacteria and/or fungi from the feet of the customer, the hands of the pedicurist, and/or from the air may remain on the surface of the tub even after the tub is thoroughly rinsed with clean water. In some pedicure spas, a gel coating or other coating is applied to the surface of the tub to enhance the appearance and durability of the tub, but such a coating does not have any antibacterial function. Therefore, regardless of whether there is a coating on the surface of the tub, there is a risk of infection for pedicurists and/or customers. In addition, the remaining bacteria and/or fungi may generate unpleasant or bad odors. Although detergents or chemical sterilizers may be applied to the surface of the tub after each pedicure session to substantially reduce the risk of infection and/or bad odors, these measures are generally expensive and labor-intensive.

### SUMMARY OF THE INVENTION

Therefore, it is an object of the present invention to provide a pedicure spa with antibacterial function so that the risk of infection and/or bad odors is substantially reduced.

It is another object of the present invention to provide a pedicure spa with antibacterial function, which antibacterial function is simply and inexpensively obtained.

It is yet another object of the present invention to provide a pedicure spa with antibacterial function, which antibacterial function is reliable and remains effective for a relatively long period of time.

It is yet another object of the present invention to provide an antibacterial gel coating for top-coating a surface on a tub of a pedicure spa.

A pedicure spa with antibacterial function according to an embodiment of the present invention includes a tub having a surface and an antibacterial gel coating applied on at least a portion of the surface of the tub. The antibacterial gel coating comprises an industrially applicable gel coating (with no antibacterial function) mixed with silver nano-particles such that the concentration of silver nano-particles is approximately 50 - 200 ppm. The industrially applicable gel coating and the silver nano-particles may be mixed in a drum of an industrial mixer. The silver nano-particles are particles made of substantially pure silver (e.g., 99.9% pure silver) and have a diameter in the range of approximately 10 - 35 nm.

Instead of being mixed with a gel coating, the silver nano-particles may also be mixed into another type of coating such as a paint or enamel. Furthermore, the silver nano-particles may alternatively be incorporated directly into the material of the tub.

These and other objects and features of the present invention will become apparent from the following detailed description considered in conjunction with the accompanying drawing. It is to be understood, however, that the drawing is designed solely for purposes of illustration and not as a definition of the limits of the invention, for which reference should be made to the appended claims. It should be further understood that the drawing is not necessarily drawn to scale and that, unless otherwise indicated, it is merely intended to conceptually illustrate the structures and procedures described herein.

### BRIEF DESCRIPTION OF THE DRAWING

In the drawings:

Fig. 1 is a perspective view of a pedicure spa with antibacterial function in accordance with the present invention, in which part of a gel coating on the surface of the tub of the pedicure spa is removed to show the surface of the tub; and

Fig. 2 is a sectional view of a gel coating on a surface of the tub of Fig. 1.

### DETAILED DESCRIPTION OF THE PRESENTLY PREFERRED EMBODIMENTS

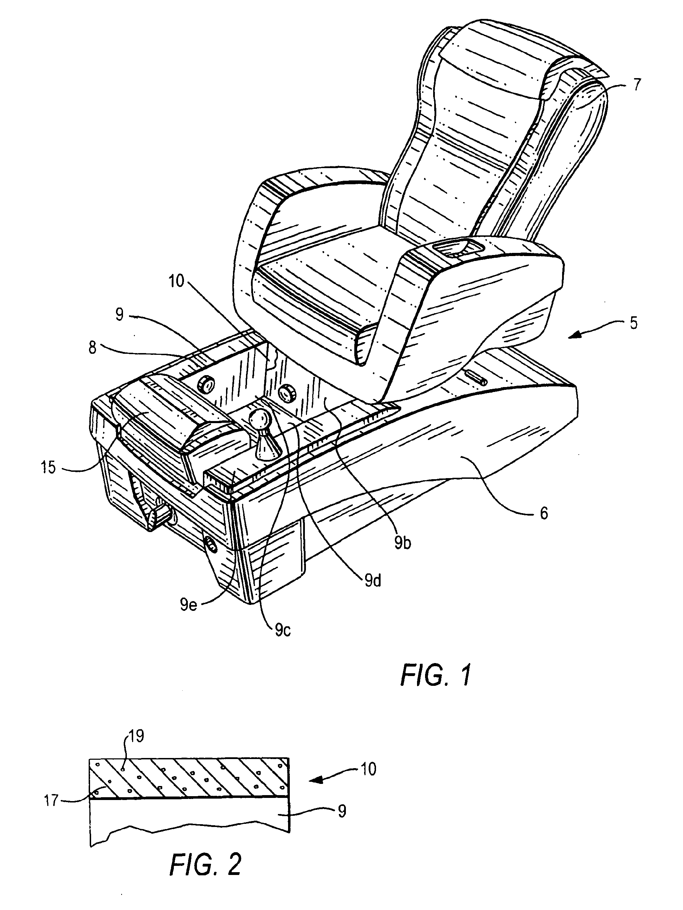

A pedicure spa 5 with antibacterial function in accordance with an embodiment of the present invention is shown in Fig. 1. The pedicure spa 5 includes a base portion 6 suitable to be placed on a floor, a chair 7 (preferably adjustable) mounted on the base portion 6, a tub or sink 8 in the base portion 6, and a footrest 15 (preferably adjustable) mounted on the base portion 6.

The tub 8 is disposed between the chair 6 and the footrest 15, and includes a surface 9. The tub 8 has a generally rectangular cross-section when viewed from above. Therefore, the surface 9 of the tub 8 includes a front side surface (not shown), a back side surface 9b, two lateral side surfaces 9c, a bottom surface 9d, and a top peripheral edge 9e. Although a rectangular-shaped tub is disclosed, the tub may comprise any suitable size and shape.

To prevent infection among customers and pedicurists and generation of bad odors on the surface 9, an antibacterial gel coating 10 is applied on the surface 9 of the tub 8, preferably covering the entire surface 9. The antibacterial gel coating 10 then cures, dries out, or hardens, and preferably remains on the surface 9 of the tub 8 during the operating life of the pedicure spa 5.

Referring to Fig. 2, the antibacterial gel coating 10 includes an industrially applicable gel 17, to which silver nano-particles 19 having a diameter in the range of approximately 10 - 35 nm are added so that the concentration of silver nano-particles is at least approximately 50 ppm and preferably in the range of 50 - 200 ppm. The antibacterial gel coating 10 can be obtained by mixing a predetermined amount of the industrially applicable gel coating 17, such as a gel coating manufactured by a Korean company, Cray Valley Korea, having an address at 821-1 Wanjoo Saneub Complex, Bong Dong Eub, Wanjoo City, Cholla-Do, Republic of Korea, with an appropriate amount of silver nano-particles 19 having a diameter in the range of approximately 10 - 35 nm, in a drum of an industrial mixer. The silver nano-particles 19 added to the industrial applicable gel coating 17 may be in colloid form.

Silver is highly toxic to bacteria and/or fungi, but it is relatively nontoxic to human cells. Silver has been used to fight infections and control spoilage since at least the times of ancient Greece and Rome. When silver particles are reduced to a nano size, such as 25 nm, they exhibit a large relative surface area, which increases their contact with bacteria and/or fungi and substantially improves their bactericidal and/or fungicidal effectiveness.

### EXAMPLES

The following tables show the bactericidal effectiveness of an antibacterial gel coating 10 that contains approximately 100 - 200 ppm of silver nano-particles having a diameter of approximately 25 nm:

**Table 1**

| | | A gel coating without silver nano- particles | The gel coating with 100 ppm of silver nano-particles having a diameter of approximately 25 nm |
|---|---|---|---|
| Bacteria-1 | At Beginning | 3.7 × 10⁵ | 3.7 × 10⁵ |
| | After 24 hrs | 1.0 × 10⁵ | <10 |
| | Value of Anti-Microbial Activity | --- | 4.0 |
| | Percent Reduction of Bacteria | --- | 99.9 |
| Bacterial-2 | At Beginning | 2.5 × 10⁵ | 2.5 × 10⁵ |
| | After 24 hrs | 7.0 × 10⁶ | <10 |
| | Value of Anti-Microbial Activity | --- | 5.8 |
| | Percent Reduction of Bacteria | --- | 99.9 |

**Table 2**

| | | A gel coating without silver nano-particles | The gel coating with 150 ppm of silver nano-particles having a diameter of approximately 25 nm |
|---|---|---|---|
| Bacteria-1 | At Beginning | 3.7 × 10⁵ | 3.7 × 10⁵ |
| | After 24 hrs | 1.0 × 10⁵ | <10 |
| | Value of Anti-Microbial Activity | --- | 4.0 |
| | Percent Reduction of Bacteria | --- | 99.9 |
| Bacterial-2 | At Beginning | 2.5 × 10⁵ | 2.5 × 10⁵ |
| | After 24 hrs | 7.0 × 10⁶ | <10 |
| | Value of Anti-Microbial Activity | --- | 5.8 |
| | Percent Reduction of Bacteria | --- | 99.9 |

**Table 3**

| | | A gel coating without silver nano-particles | The gel coating with 200 ppm of silver nano-particles having a diameter of approximately 25 nm |
|---|---|---|---|
| Bacteria-1 | At Beginning | 3.7 × 10⁵ | 3.7 × 10⁵ |
| | After 24 hrs | 1.0 × 10⁵ | <10 |
| | Value of Anti-Microbial Activity | --- | 4.0 |
| | Percent Reduction of Bacteria | --- | 99.9 |
| Bacterial-2 | At Beginning | 2.5 × 10⁵ | 2.5 × 10⁵ |
| | After 24 hrs | 7.0 × 10⁶ | <10 |
| | Value of Anti-Microbial Activity | --- | 5.8 |
| | Percent Reduction of Bacteria | --- | 99.9 |

The following note is applicable to each of Tables 1-3:

Antimicrobial activity and efficacy (film-contact method, JIS Z 2801-2000): Cells/^{mℓ}, Value of Antimicrobial Activity: log, Percent Reduction of Bacteria: %

| | |
|---|---|
| Standard Film: | Stomacher 400 Poly-Bag |
| Test Condition: | The solution is fixed at 35 ±1 °C, RH 90±5% for 24 hrs, and determine bacterial cell growth inhibition rate by pour agar plate method |
| Test Bacteria: 6538P | Bacteria-1 - *Staphylococcus aureus* ATCC |
| | Bacteria-2 - *Escherichia coli* ATCC 8739 |

Various modifications of the basic structures, functionality and steps described above are within the intended scope and contemplation of the invention. For example, the antibacterial gel coating 10 may be applied on the entire bottom surface 9d, but only on the lower portions of the front side surface, the back side surface 9b, and the two lateral side surfaces 9c. In addition, instead of using an industrially applicable gel 17, an industrially applicable paint, enamel, or other surface covering known or thereafter developed may be mixed with silver nano-particles. Furthermore, the silver nano-particles may be incorporated directly into the surface of the tub material.

While there have shown and described and pointed out fundamental novel features of the invention as applied to a preferred embodiment thereof, it will be understood that various omissions and substitutions and changes in the form and details of the device illustrated, and in its operation, may be made by those skilled in the art without departing from the spirit of the invention. For example, it is expressly intended that all combinations of those elements which perform substantially the same function in substantially the same way to achieve the same results are within the scope of the invention. Moreover, it should be recognized that structures and/or elements shown and/or described in connection with any disclosed form or embodiment of the invention may be incorporated in any other disclosed or described or suggested form or embodiment as a general matter of design choice.

## Claims

1. A pedicure spa with antibacterial function, comprising:
a base support for supporting the pedicure spa on an underlying surface;
a chair arranged on said base support;
a tub arranged on said base support and having a surface defining a space for receiving a foot of a customer when the customer is sitting in said chair; and
an antibacterial coating on at least a portion of the surface of the tub and comprising silver nano-particles having a diameter in the range of approximately 10 - 35 nm.

2. The pedicure spa of claim 1, wherein the antibacterial coating further comprises an industrially applicable coating mixed with the silver nano-particles so that a concentration of silver nano-particles in the antibacterial coating is at least approximately 50 ppm and applied to the at least a portion of the surface of the tub.

3. The pedicure spa of one of the preceding claims, wherein the antibacterial coating contains approximately 50 - 200 ppm of the silver nano-particles.

4. The pedicure spa of one of claims 1 and 2, wherein the antibacterial coating contains approximately 50 - 100 ppm of the silver nano-particles.

5. The pedicure spa of one of claims 1 and 2, wherein the antibacterial coating contains approximately 100 - 200 ppm of the silver nano-particles.

6. The pedicure spa of one of the preceding claims, wherein the silver nano-particles have a diameter of approximately 25 nm.

7. The pedicure spa of one of claims 1, 2, 4 and 6, wherein the antibacterial coating contains approximately 50 - 100 ppm of the silver nano-particles having a diameter of approximately 25 nm.

8. The pedicure spa of claim 2, wherein the industrially applicable coating is a gel coating.

9. An antibacterial gel coating for top-coating at least a portion of an object, wherein the antibacterial gel coating includes an industrially applicable gel coating mixed with silver nano-particles having a diameter in the range of approximately 10 - 35 nm such that a concentration of the silver nano-particles is at least approximately 50 ppm.

10. The antibacterial gel coating of claim 9, wherein the antibacterial gel coating contains approximately 50 - 200 ppm of the silver nano-particles.

11. The antibacterial gel coating of claim 9, wherein the antibacterial gel coating contains approximately 50 - 100 ppm of the silver nano-particles.

12. The antibacterial gel coating of claim 9, wherein the antibacterial gel coating contains approximately 100-200 ppm of the silver nano-particles.

13. The antibacterial gel coating of one of claims 9 to 12, wherein the silver nano-particles have a diameter of approximately 25 nm.
